# EUROPEAN PATENT APPLICATION

(11) **EP 3 578 144 A1**
(43) Date of publication of application: **11.12.2019**
(21) Application number: 17889180.0
(22) Date of filing: 14.04.2017
(51) Int. Cl.: A61F 2/95

(54) **DEVICE AND METHOD FOR SAFELY POSITIONING A CORONARY STENT IN THE CORONARY ARTERIES**

(30) Priority: 30.12.2016 RU 2016152736
(71) Applicant: Seven Sons Ltd. R.N. 515985570 (The "Company"), 67443 Tel Aviv (IL)
(72) Inventor: SARANIN, Sergei Alexandrovich, Serpukhov - Moskovskaya obl. 142204 (RU); KOBAK, Anton Alexandrovich, St. Petersburg 193232 (RU)
(74) Representative: Loven, Keith James
(86) International application number: PCT/RU2017/000236
(87) International publication number: WO 2018/124925

(57) **Abstract**

The present invention is suitable for use in medicine to enable a maximally accurate, fast and safe positioning of a coronary stent in case of uncomplicated and complicated anatomic lesions of the coronary bed as well as for stenting renal, visceral arteries. The claimed device comprises the housing having the truncated front part and the cylindrical rear part with the thread on the internal side of the surface, the gripping means with flaps, the pressure protrusion on one side and the protrusion on the gripping means guides, the release mechanism with the pressure surface, flaps and the gripping means in the front part of the housing. In the preparation for use, a coronary stent carried by the delivery system is in advance progressively inserted inside the device following which the coronary stent carried by the delivery system is manually advanced to the affected area of the coronary artery, the delivery system is fixed in the device by the gripping means flaps, and then by rotating circumferentially the cylindrical rear part of the device, the stent is positioned within the coronary artery.

## Description

The present invention is intended for use in medicine in order to enable a maximally accurate, fast and safe positioning of a coronary stent in case of uncomplicated and complicated anatomic lesions of the coronary bed, in particular within the coronary artery in case of endovascular surgery by means of coronary stenting for recanalization of the artery portions constricted as a result of lesion.

The device is intended for use in case of coronary ostial lesions, coronary bifurcation lesions, including lesions of the left coronary artery trunk and ostial lesions of the right coronary artery, as well as in case of "stent-to-stent" implantation for reducing the "overlap" area.

The use of this device allows the radiation exposure of patients and medical personnel as well as the exposure of patients to radio-opaque contrast agents to be significantly reduced.

The device may be successfully used by young professionals at the time of their professional development in the field of interventional cardiology.

In addition, the claimed device may be also used for stenting renal, visceral arteries in a similar way.

A number of devices for positioning a stent within the coronary artery are available at the medical equipment market.

In particular, the Patent No. US 6293964 discloses OSTIAL PRO available from Merit Medica (USA). The device according to this patent comprises a cone made up of four tongues insertable into a guiding catheter together with a coronary stent. The guiding catheter is selectively positioned within the coronary ostium. The coronary stent is guided along the catheter to a location more distal that the coronary ostium whereafter the guiding catheter is withdrawn from the coronary ostium and the device is deployed. As the device leaves the guiding catheter, its tongues unfold and abut against the aortal wall so that the guiding catheter tip cannot be selectively positioned within the coronary ostium as required for ostial stenting of the left coronary artery trunk and the right coronary artery trunk. The entire subsequent positioning of the coronary stent within the affected area is performed manually.

A disadvantage of the known device is its limited applicability, in particular only in cases of ostial lesions of the left coronary artery trunk and the right coronary artery trunk, as well as the manual positioning of the coronary stent.

Also known is a device for a safe positioning of a coronary stent within coronary arteries according to the Patent of the Russian Federation No. 2598798 owned by Seven Sons OJSC.

The claimed device differs primarily in:
- faster and more accurate positioning of a coronary stent within coronary artery,
- mechanical positioning rather than manual positioning of the stent,
- reduced radiation exposure of patients and medical personnel due to reduced time of the coronary stent positioning,
- applicability of the device in case of lesions within any segment of the coronary bed,
- reduced exposure of patients to radio-opaque contrast agents due to reduced time of the coronary stent positioning,
- single applicability,
- the device is conveniently configured as extension of the surgeon's arm,
- minimal weight and dimensional parameters,
- ease of use,
- cost-effective production.

The device is industrially applicable.

The claimed device has a housing comprising two halves: a truncated front part and a cylindrical rear part made in the shape of a glass. The housing is formed of plastic by injection molding, wherein its surface contacting the operator's arm is pebbled. The housing is provided with a horizontally extending hole for receiving a coronary stent delivery system, the truncated front part of the housing has recesses on its both sides for the operator's left thumb and left forefinger to hold the device fixed in position, wherein the recess for the left thumb also serves as a pad for fixing the coronary guide. The cylindrical rear part is connected to the front part in such a way that it can rotate around its axis. The front and rear parts of the housing have holes for insertion of the coronary stent delivery system into the housing.

On the inner surface of the cylindrical rear part of the housing, a thread is circumferentially arranged contacting a protrusion formed on one side of a runner arranged on guides of the front part of the housing and horizontally displaceable along guides inside the housing.

The gripping means have two flaps connected to each other at the point of fixing the gripping means to the body of the gripping means, which are horizontally displaceable inside the housing; one is stiff and the other is elastic and has an edge.

The gripping means allow to fix the coronary stent delivery system rigidly.

A release means is arranged in the truncated front part of the housing, comprising a pressure surface on an outer side of the truncated front part of the housing, and flaps arranged inside the housing.

When pressure is exerted to the pressure surface arranged on an outer side of the housing, the release means flaps press onto the pressure roller which causes the protrusions on the gripping means flaps to contract so that the flaps with elastic members at the gripping point of the delivery system that are arranged inside the bushing freely rotatably around its axis open to set free a through hole in the gripping means for placing the coronary stent delivery system inside the device following which the pressure surface is released, the gripping means flaps, the pressure roller and the protrusions on the gripping means flaps return to their initial position and the flaps with elastic members fix the delivery system, the operator causes the cylindrical rear part of the housing to rotate circumferentially with his/her right arm for advancing the delivery system longitudinally to a necessary distance forwards or backwards along the guides at the operator's discretion due to engagement of the runner protrusion with the internal thread of the cylindrical rear part of the housing.

Once the delivery system is placed in position, the rotation of the cylindrical rear part of the housing is stopped.

In this case, if the operator wants to return the delivery system into the initial position and continue to work, he/she presses on the release mechanism, releases the delivery system and rotates the rear part of the housing thereby returning the release mechanism into the initial position, releases the mechanism and continues working. In this way, when the release mechanism is in a pressure-free state after the flaps with elastic members contract at the gripping point of the delivery mechanism on the bushing side, the delivery system coronary stent is held fixed, and when pressure is exerted on the pressure surface after contracting the pressure roller that exerts pressure on the protrusions on the gripping means flaps and causes the flaps with elastic members to open on the system gripping side, the delivery system coronary stent is released.

In the preparation for use, a coronary stent carried by the delivery system is first progressively inserted inside the device to which end the operator exerts pressure on the release means pressure surface, the release means flaps cause the pressure roller to contract, which in turn causes the protrusions arranged inside the roller on the gripping means flaps to contract and to open the flaps of the gripping means with elastic members at the gripping point of the delivery system on the bushing side so that the operator, through the hole in the cylindrical rear part of the housing, through the hole being formed between the gripping means flaps and also through the hole in the truncated front part of the housing, places the coronary stent delivery system inside the housing of the device. When no pressure is exerted on the outer pressure surface of the release means, the flaps no longer exert pressure on the pressure roller, the protrusions on the flaps within the pressure roller unfold, the flaps of the gripping means with elastic members contract at the gripping point of the delivery system on the bushing side and hold the delivery system. The operator manually advances the coronary stent carried by the delivery system placed within the device to the affected area of the coronary artery. Then, pressure is again exerted on the outer pressure surface of the release mechanism, the pressure roller causes the protrusions on the flaps to contract, the flaps open at the gripping point of the delivery system on the bushing side, and the operator brings the device to a necessary distance for fixing the same with his/her left arm, releases the pressure surface, and rotates the cylindrical rear part of the housing with his/her right arm to convert the rotary motion of the rear part of the housing into longitudinal translational motion of the runner with the gripping means along the guides inside the housing to displace the delivery system to a necessary distance for positioning of the coronary stent.

When exerting pressure on the pressure surface of the release means, the operator may also return the mechanism fixing the coronary stent delivery system into its initial position by rotating the cylindrical rear part of the housing in the necessary direction so that further positioning of the coronary stent forwards or backwards in each of the directions may be continued.

In an embodiment, the device may be already provided with the delivery system fixed therein. In this case, the stage of inserting in advance the coronary stent carried by the delivery system inside the device is omitted. The coronary stent carried by the delivery system is manually advanced to the affected area of the coronary artery and then pressure is exerted on the pressure surface, the flaps unfold at the gripping point of the delivery system, and the operator brings the device to a necessary distance for fixing the same with his/her left arm, releases the pressure surface, and rotates the cylindrical rear part of the housing with his/her right arm to displace progressively the delivery system to a necessary distance for positioning the coronary stent within the artery. When pressure is exerted on the pressure surface, by fixing the delivery system, the operator may also return to initial position the mechanism fixing the coronary stent delivery system by rotating the cylindrical portion of the housing in the necessary direction so that further positioning of the coronary stent forwards and backwards may be continued within the dimensions of the guides inside the housing in each of the directions.

In an embodiment, for a safe placing of the stent on the delivery system, a hollow tube prearranged inside the device may be used for inserting therein the delivery system carrying the stent following which the tube is removed from the device.

Listed below are the attached drawings wherein:

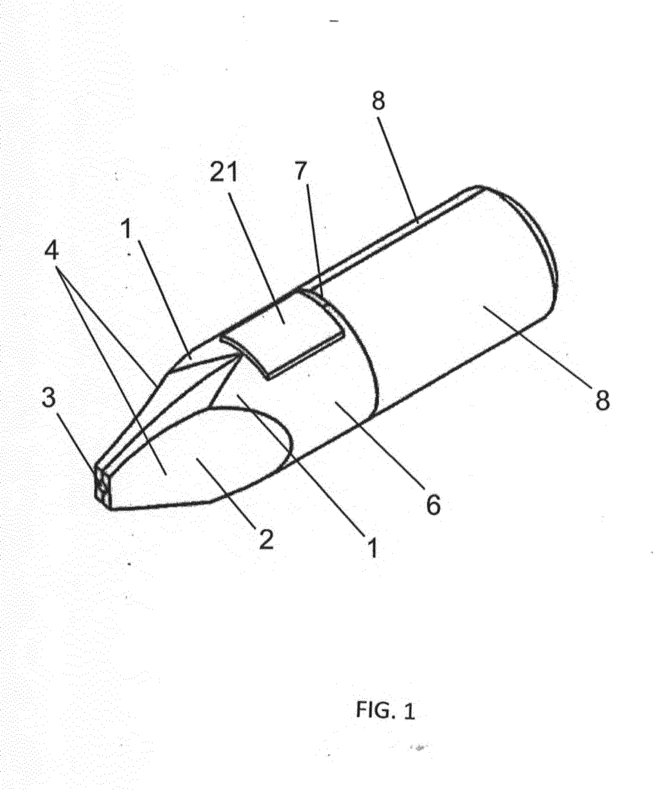

**Fig. 1** is the general view of the device, showing:
   1 - device housing,
   2 - truncated front part of the housing,
   3 - hole in the truncated front part of the housing for the delivery system,
   4 - recess for left arm fingers,
   6 - cylindrical portion of the front part of the housing,
   7 - channel for connection of the front part of the housing and the rear cylindrical part of the housing,
   8 - rear cylindrical part of the housing,
   21 - release mechanism pressure surface.

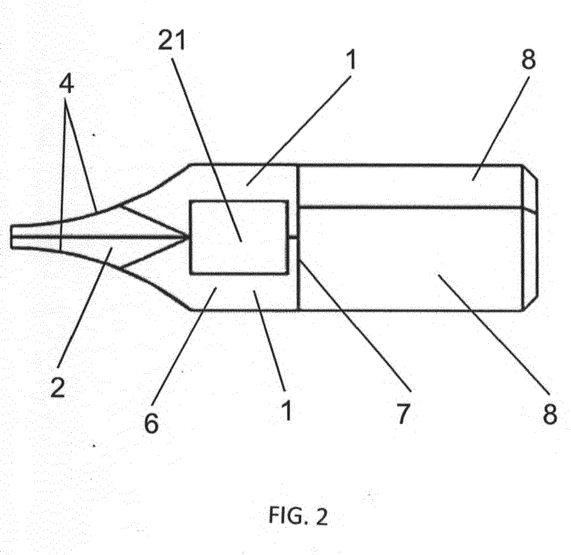

**Fig. 2** is the top view, showing:
   1 - device housing,
   2 - truncated front part of the housing,
   4 - recess for left arm fingers,
   6 - cylindrical portion of the front part of the housing,
   7 - channel for connection of the front part of the housing and the rear cylindrical part of the housing,
   8 - rear cylindrical part of the housing,
   21 - release mechanism pressure surface.

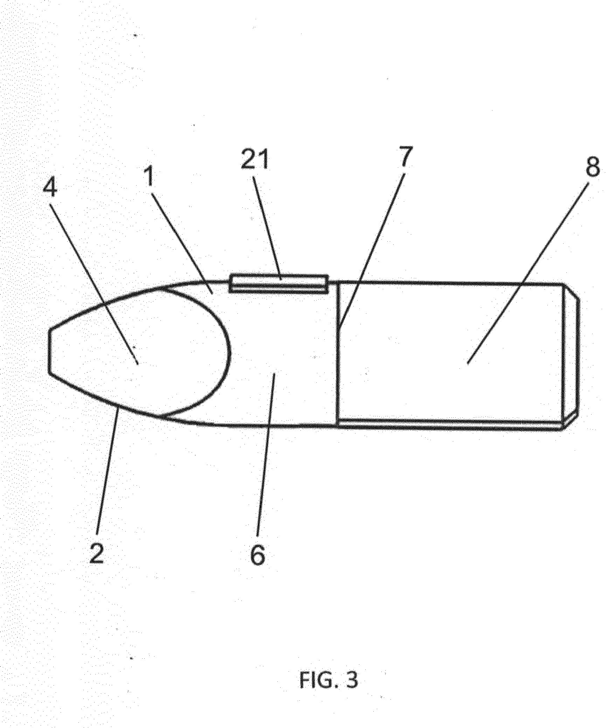

**Fig 3** is the side view, showing:
   1 - device housing,
   2 - truncated front part of the housing,
   4 - recess for left arm fingers,
   6 - cylindrical portion of the front part of the housing,
   7 - channel for connection of the front part of the housing and the rear cylindrical part of the housing,
   8 - rear cylindrical part of the housing,
   21 - release mechanism pressure surface.

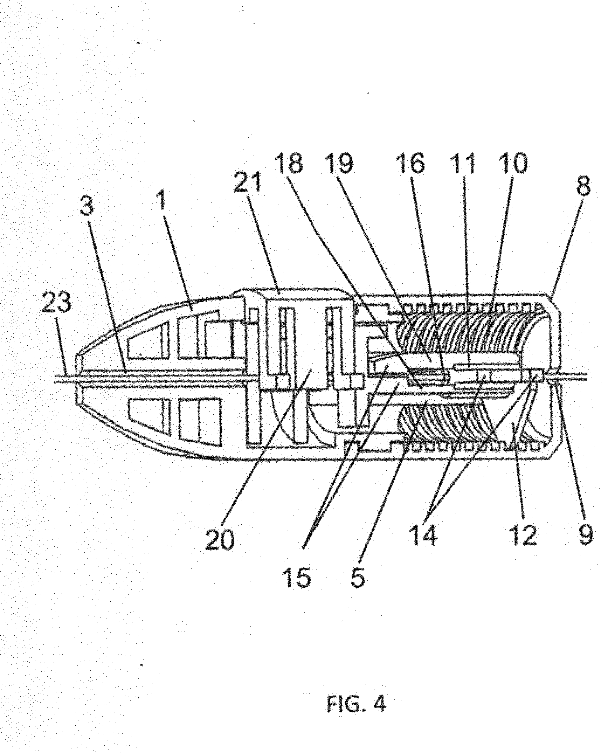

**Fig. 4** is the cross-sectional side view, showing:
   1 - device housing,
   3 - hole in the truncated front part of the housing for the delivery system,
   4 - recess for left arm fingers,
   **5** - **cut for movement of the gripping means inside the housing,**
   8 - rear cylindrical part of the housing,
   9 - hole in the cylindrical rear part of the housing for the delivery system,
   10 - internal thread in the cylindrical rear part,
   11 - gripping means,
   12 - gripping means protrusion to connect with the thread of the cylindrical rear part of the housing,
   14 - gripping means guides,
   15 - elastic elements on gripping means flaps,
   16 - hole in the gripping means to place the delivery system,
   18 - elastic gripping means flap,
   19 - rigid gripping means flap,
   20 - release mechanism,
   21 - release mechanism pressure surface,
   23 - delivery system.

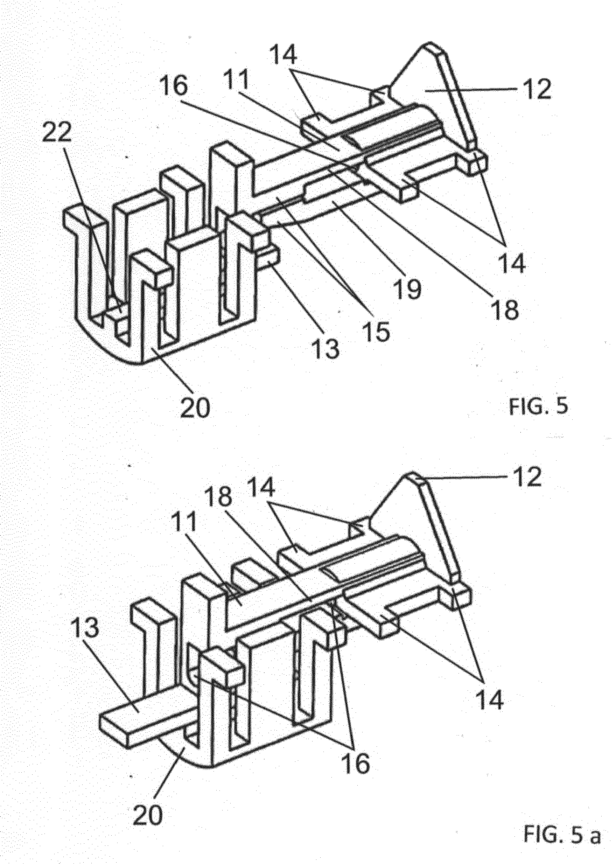

**Fig. 5** is the bottom view of the delivery mechanism with closed release mechanism flaps assembled, showing:
   11 - gripping means,
   12 - gripping means protrusion to connect with the thread of the cylindrical rear part of the housing,
   13 - elastic flap protrusion for opening the gripping means,
   14 - gripping means guides,
   15 - elastic elements on gripping means flaps,
   16 - hole in the gripping means to place the delivery system,
   18 - elastic gripping means flap,
   19 - rigid gripping means flap,
   20 - release mechanism,
   22 - flaps of the release mechanism.
**Fig. 5a** is the bottom view of the delivery mechanism with open release mechanism flaps assembled, showing:
   11 - gripping means,
   12 - gripping means protrusion to connect with the thread of the cylindrical rear part of the housing,
   13 - elastic flap protrusion for opening the gripping means,
   14 - gripping means guides,
   16 - hole in the gripping means to place the delivery system,
   18 - elastic gripping means flap,
   20 - release mechanism.

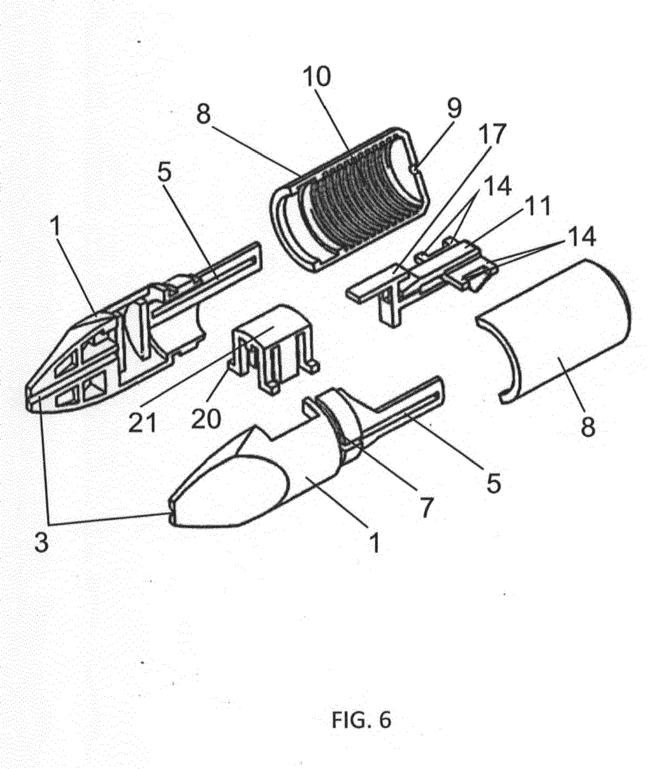

**Fig. 6** is the view of the disassembled device, showing:
   1 - device housing,
   3 - hole in the truncated front part of the housing for the delivery system,
   **5** - cut for movement of the gripping means inside the housing,
   7 - channel for connection of the front part of the housing and the rear cylindrical part of the housing,
   8 - rear cylindrical part of the housing,
   9 - hole in the cylindrical rear part of the housing for the delivery system,
   10 - internal thread in the cylindrical rear part of the housing,
   11 - gripping means,
   14 - gripping means guides,
   17 - gripping means pressure surface for opening flaps,
   20 - release mechanism,
   21 - release mechanism pressure surface.

The invention will be described below with reference to the attached drawings.

Invention embodiment, example 1 (Fig. 1), formed of plastic by injection molding, wherein its surface is pebbled where it contacts the operator's arm 4 (Fig. 1), and the housing left and right halves (Fig. 1) are manufactured separately and assembled after placing therein gripping means 11 (Fig. 4, Fig. 5, Fig. 5a, Fig. 6), consisting of the elastic flap 18 with protrusion 13 (Fig. 5a) to adhere to the pressure surface 17 (Fig. 6) and rigid flap 19 (Fig. 5, Fig. 6) with protrusion 12 (Fig. 4, Fig. 5, Fig. 5a) on one side of the gripping means with internal thread 10 (Fig. 4, Fig. 6) rear part 8 of housing 1 (Fig. 1, Fig. 4) and placed on the side of the pressure surface 17 (Fig. 6), designated for opening the elastic flap 18 (Fig. 4, Fig. 5, Fig. 5a) gripping means 11 (Fig. 4, Fig. 5, Fig. 5a, Fig. 6) and the release mechanism 20 (Fig. 4, Fig. 5, Fig. 5a, Fig. 6), comprising the pressure surface 21 (Fig. 1, Fig. 2, Fig. 3, Fig. 4, Fig. 5), placed on the outer side of the housing 1 (Fig. 1, Fig. 4) and flaps 22, designated for interaction with the pressure surface 17 of the gripping means 11 (Fig. 5, Fig. 6).

The housing has a truncated front part 2 (Fig. 1, Fig. 2, Fig. 3) with a through hole 3 (Fig. 4) for the delivery system 23 (Fig 4) and a recess for left arm's fingers 4 (Fig. 1, Fig. 2, Fig. 3), cylindrical rear part 8 (Fig. 1, Fig. 2, Fig. 3, Fig. 4) with a through hole 9 (Fig. 4) for the delivery system 23 (Fig. 4). There is pressure surface 21 (Fig. 1, Fig. 2, Fig. 3) of the release mechanism 20 (Fig. 4, Fig. 5, Fig. 6) in the upper part of the housing 1 (Fig. 1). The truncated front part 2 (Fig. 1, Fig. 2, Fig. 3) of the housing 1 (Fig. 1, Fig. 2, Fig. 3) is connected to the cylindrical rear part of the housing 8 (Fig. 1, Fig. 2, Fig. 3) with the channel 7 (Fig. 6). There are channels 5 (Fig. 6) inside the housing for the gripping means guides.

The mechanism placed in the housing 1 (Fig. 1, Fig. 2, Fig. 3), includes gripping means 11 (Fig. 4, Fig. 5, Fig. 5a, Fig. 6), consisting of the elastic flap 18 (Fig. 4, Fig. 5, Fig. 5a) with protrusion 13 (Fig. 5, Fig. 5a), designated for contacting the pressure surface 17 (Fig. 6), and rigid flap 19 (Fig. 4, Fig. 5) with elastic elements 15 on the internal surface (Fig. 4. Fig. 5), placed on guides 14 (Fig. 4, Fig. 5, Fig. 5a, Fig. 6), protrusion 12 (Fig. 4, Fig. 5), designated for connection to the internal thread 10 (Fig. 4, Fig. 6) rear part 8 (Fig. 1, Fig. 2, Fig. 4) housing 1 (Fig. 1, Fig. 2, Fig. 4), pressure surface 17 gripping means 11 (Fig. 6) to open the elastic flap 18, (Fig. 4, Fig. 5) gripping means 11 (Fig. 4, Fig. 5, Fig. 5a, Fig. 6), release mechanism 20 (Fig. 4, Fig. 5, Fig. 5a, Fig. 6), consisting of the pressure surface 21 (Fig. 1, Fig. 2, Fig. 3, Fig. 4, Fig. 6), located on the outer truncated front part 2 (Fig. 1, Fig. 2, Fig. 3, Fig. 4) housing 1 (Fig. 1, Fig. 2, Fig. 3, Fig. 4), and flaps 22 of the release mechanism (Fig. 5), contacting the protrusion 13 of the elastic flap 18 of the gripping means 11 (Fig. 5, Fig. 5a) for opening and closure of flaps 18 (Fig. 5, Fig. 5a) of the gripping means 11 (Fig. 5, Fig. 5a).

In order to place the delivery system 23 (Fig. 4) inside the device the operator using right arm's fingers presses on the pressure surface 21 (Fig. 1, Fig. 2, Fig. 3) of the release mechanism 20 (Fig. 4, Fig. 5, Fig. 5a, Fig. 6), placed in the front part 2 of the housing 1 (Fig. 4), which flaps 22 (Fig. 5) placed inside the housing on the side opposite to the pressure surface 21 (Fig. 4), when the operator presses on the pressure surface 21 (Fig. 1, Fig. 2, Fig. 3) press on protrusion 17 (Fig. 5, Fig. 6) of the gripping means 11 and using the protrusion 13 (Fig. 5, Fig. 5a) elastic flap 18 moves the flap 18 (Fig. 5), opening the through hole 16 of the gripping means 11 (Fig. 4, Fig. 5a) to insert the delivery system 23 (Fig. 4) into the housing 1 of the device. Using holes 3, 9 (Fig. 4) in the front and rear parts of the housing 1 (Fig. 4) and hole 16 between flaps 18, 19 (Fig. 4, Fig. 5a) of the gripping means 11 (Fig. 4, Fig. 5a) the delivery system 23 (Fig. 4) is inserted in the device, after which the operator releases the pressure surface 21 (Fig. 1, Fig. 2, Fig. 4) and the delivery system 23 (Fig. 4) is fixed between the flaps 18, 19 (Fig. 5) with elastic elements 15 on the internal side (Fig. 4) of the gripping means 11 (Fig. 4), and the operator using the right arm's fingers rotates the cylindrical rear part 8 of the housing 1 around the circumference (Fig. 1, Fig. 3) in the required direction transforming the rotational movement of the rear part of the housing 8 into the progressive movement of the gripping means 11 (Fig. 4), where the protrusion 12 (Fig. 4) of the gripping means 11 (Fig. 4), contacting the internal thread 10 (Fig. 4) of the cylindrical part 8 (Fig. 4) of the housing 1, moves the gripping means 11 (Fig. 4) with the delivery system 23 (Fig. 4) longitudinally inside the housing 1 at a required distance along channels 5 in the housing of the gripping means.

When the operator pushes on the pressure surface 21 (Fig. 1, Fig. 2, Fig. 3) of the release mechanism 20 (Fig. 5, Fig. 5a, Fig. 6), he/she may return the mechanism fixing the coronary stent delivery system into the initial position by rotating the cylindrical rear part 8 of the housing 1 in a required direction, which allows continuing further positioning back and forth along the guides 5 (Fig. 4, Fig. 6) inside the housing 1 in each side.

Another embodiment of the invention has the delivery system 23 (Fig. 4) prior fixed therein, where the need in prior placement of the delivery system inside the device is excluded.

Another embodiment of the invention for safe positioning of the stent in the delivery system, an empty tube may be used, which has been prior placed inside the device, in which the delivery system 23 (Fig. 4) with the stent is inserted, then the tube is removed from the device.

## Claims

1. A device for positioning of a coronary stent within coronary arteries, **characterized in that** it comprises a housing with a through hole for placing a coronary stent delivery system, said housing comprising a truncated front part with recesses for the operator's arm and a cylindrical rear part connected to the front part, rotatably relative to the front part, and provided with a circumferential thread in contact with a protrusion on guides arranged inside the cylindrical rear part so as to be displaceable along the guides inside the housing with two flaps with rubbers at the capture area of the delivery system, where **one** flap is elastic and has a protrusion, and the other is rigid; there is a release mechanism in the truncated front part of the housing, which may contact the pressure surface of the gripping means and the protrusion of the gripping means elastic flap, the outer part of which is a pressure plate placed on the outer side of the housing, and the internal part is made in the form of flaps and placed inside the housing on the side opposite to the pressure surface.

2. The device, according to example 1, is **characterized in that** the housing is formed of plastic, wherein its surface contacting the operator's arm is pebbled.

3. The device, according to example 1, is **characterized in that** the device is provided with a delivery system fixed therein in advance and carrying a stent.

4. The device, according to example 1, is **characterized in that** the device is provided with an empty tube aimed at placement of the delivery system carrying a stent.

5. A device for positioning of a coronary stent within coronary arteries, **characterized in that** when the coronary stent delivery system is placed in the housing the pressure surface of the release mechanism, which inside part placed inside the housing on the side opposite to the pressure surface, and comprising flaps contacts the gripping means pressure surface placed in the cylindrical rear part of the housing having two flaps on the outer side of the housing is pressed; one of such flaps is elastic and has a protrusion, and the other is rigid displacing the elastic flap protrusion and forming the hole, in which the delivery system is placed through holes on the front and rear sides of the housing; then the pressure surface is released, and the coronary stent delivery system is fixed inside the housing of the device, and then by rotating the cylindrical rear part of the housing along circumference relatively the front part of the housing, the delivery system is moved along at a required distance with transformation of the rotational movement of the cylindrical part of the housing into the progressive movement as a result of gripping the gripping means protrusion placed on the gripping means guides with a thread on the internal surface of the cylindrical rear part of the housing.
